# EUROPEAN PATENT APPLICATION

(11) **EP 0 535 580 A1**
(43) Date of publication of application: **07.04.1993**
(21) Application number: 92116586.6
(22) Date of filing: 28.09.1992
(51) Int. Cl.: B26F 1/26, B29C 59/04

(54) **Pressure sensitive valve system and process for forming said system**

(30) Priority: 30.09.1991 US 768782
(71) Applicant: KIMBERLY-CLARK CORPORATION, Neenah Wisconsin 54957-0349 (US)
(72) Inventor: Jameson, Lee Kirby, Roswell, Georgia 30075 (US); Cohen, Bernard, Berkley Lake, Georgia 30136 (US)
(74) Representative: Grünecker, Kinkeldey, Stockmair & Schwanhäusser Anwaltssozietät

(57) **Abstract**

A thin elastomeric polymeric sheet material having at least about 100 valves per square inch is disclosed. In some embodiments the valves are microvalves where the maximum functional aperture of each of the microvalves generally ranges from greater than about 10 square micrometers to less than about 100,000 square micrometers. Also disclosed is a one-way valve system.

## Description

### RELATED APPLICATIONS

This application is one of a group of applications which are being filed on the same date. It should be noted that this group of applications includes U.S. patent application serial number entitled "Hydrosonically Microapertured Thin Thermoset Sheet Materials" in the names of Lee K. Jameson and Bernard Cohen; U.S. patent application serial number entitled "Hydrosonically Microapertured Thin Thermoplastic Sheet Materials" in the names of Bernard Cohen and Lee K. Jameson; U.S. patent application serial number entitled "Pressure Sensitive Valve System and Process For Forming Said System" in the names of Lee K. Jameson and Bernard Cohen; U.S. patent application serial number entitled " Hydrosonically Embedded Soft Thin Film Materials and Process For Forming Said Materials" in the names of Bernard Cohen and Lee K. Jameson; U.S. patent application number entitled "Hydrosonically Microapertured Thin Naturally Occurring Polymeric Sheet Materials and Method of Making the Same" in the names of Lee K. Jameson and Bernard Cohen; U.S. patent application serial number entitled "Hydrosonically Microapertured Thin Metallic Sheet Materials" in the names of Bernard Cohen and Lee K. Jameson; U.S. patent application serial number entitled "Process For Hydrosonically Microaperturing Thin Sheet Materials" in the names of Lee K. Jameson and Bernard Cohen; and U.S patent application serial number entitled "Process For Hydrosonically Area Thinning Thin Sheet Materials" in the names of Bernard Cohen and Lee K. Jameson. All of these applications are hereby incorporated by reference.

### FIELD OF THE INVENTION

The field of the present invention encompasses pressure sensitive valves.

### BACKGROUND OF THE INVENTION

Ultrasonics is basically the science of the effects of sound vibrations beyond the limit of audible frequencies. Ultrasonics has been used in a wide variety of applications. For example, ultrasonics has been used for (1) dust, smoke and mist precipitation; (2) preparation of colloidal dispersions; (3) cleaning of metal parts and fabrics; (4) friction welding; (5) the formation of catalysts; (6) the degassing and solidification of molten metals; (7) the extraction of flavor oils in brewing; (8) electroplating; (9) drilling hard materials; (10) fluxless soldering and (10) nondestructive testing such as in diagnostic medicine.

The object of high power ultrasonic applications is to bring about some permanent physical change in the material treated. This process requires the flow of vibratory power per unit of area or volume. Depending on the application, the power density may range from less than a watt to thousands of watts per square centimeter. Although the original ultrasonic power devices operated at radio frequencies, today most operate at 20-69 kHz.

The piezoelectric sandwich-type transducer driven by an electronic power supply has emerged as the most common source of ultrasonic power; the overall efficiency of such equipment (net acoustic power per electric-line power) is typically greater than 70%. The maximum power from a conventional transducer is inversely proportional to the scare of the frequency. Some applications, such as cleaning, may have many transducers working into a common load.

Other, more particular areas where ultrasonic vibratory force has been utilized are in the areas of thin nonwoven webs and thin films. For example, ultrasonic force has been use to bond or weld nonwoven webs. See, for example, U.S. patent numbers 3,575,752 to Carpenter, 3,660,186 to Sager et al., 3,966,519 to Mitchell et al. and 4,695,454 to Sayovitz et al. which disclose the use of ultrasonics to bond or weld nonwoven webs. U.S. patent numbers 3,488,240 to Roberts, describes the use of ultrasonics to bond or weld thin films such as oriented polyesters.

Ultrasonic force has also been utilized to aperture nonwoven webs. See, for example, U.S. patent numbers 3,949,127 to Ostermeier et al. and 3,966,519 to Mitchell et al..

Lastly, ultrasonic force has been used to aperture thin film material. See, for example, U. S. patent number 3,756,880 to Graczyk.

Other methods for the aperturing of thin film have been developed. For example, U.S. patent number 4,815,714 to Douglas discusses the aperturing of a thin film by first abrading the film, which is in filled and unoriented form, and then subjecting the film to corona discharge treatment.

One of the difficulties and obstacles in the use of ultrasonic force in the formation of apertures in materials is the fact that control of the amount of force which is applied was difficult. This lack of control generally resulted in the limitation of ultrasonic force to form large apertures as opposed to small microapertures. Such an application is discussed in U.K. patent application number 2,124,134 to Blair. One of the possible reasons that ultrasonics has not found satisfactory acceptance in the area of microaperture formation is that the amount of vibrational energy required to form an aperture often resulted in a melt-through of the film.

As has previously been stated, those in the art had recognized that ultrasonics could be utilized to form apertures in nonwoven webs. See, U.S patent to Mitchell. et al.. Additionally, the Mitchell et al. patent discloses that the amount of ultrasonic energy being subjected to a nonwoven web could be controlled by applying enough of a fluid to the area at which the ultrasonic energy was being applied to the nonwoven web so that the fluid was present in uncombined form. Importantly, the Mitchell, et al. patent states that the fluid is moved by the action of the ultrasonic force within the nonwoven web to cause aperture formation in the web by fiber rearrangement and entanglement. The Mitchell et al. patent also states that, in its broadest aspects, since these effects are obtained primarily through physical movement of fibers, the method of their invention may be utilized to bond or increase the strength of a wide variety of fibrous webs.

While the discovery disclosed in the Mitchell et al. patent, no doubt, was an important contribution to the art, it clearly did not address the possibility of aperturing thin nonfibrous elastomeric polymeric sheet materials. This fact is clear because the Mitchell et al. patent clearly states the belief that the mechanism of aperture formation depended upon fiber rearrangement. Of course, thin nonfibrous elastomeric polymeric sheet materials do not have fibers and thus there are no fibers to be rearranged. Accordingly, it can be stated with conviction that the applicability of a method for aperturing thin nonfibrous elastomeric polymeric sheet materials by the application of ultrasonic energy in conjunction with a fluid at the point of application of the ultrasonic energy to the thin nonfibrous elastomeric sheet material was not contemplated by the Mitchell et al. patent. Moreover, the Mitchell et al. patent teaches away from such an application because the patent states the belief that aperture formation requires the presence of fibers to be rearranged.

Another area of interest to those of skill in the art is that of the formation of pressure sensitive valves. Pressure sensitive valves and their uses are well known and virtually too numerous to list. One area of interest is the area of pressure sensitive valves and, in particular, pressure sensitive microvalves. Microvalves are valves which, when opened in response to an application of pressure, have a maximum opening on the order of 100,000 square micrometers or less. Those in the art have been searching for a method of forming cheap, inexpensive microvalves and, of course, the microvalves themselves. It is believed that such investigations have heretofor been generally unsuccessful.

### DEFINITIONS

The term "elastomeric" is used herein to designate any relaxed, unbiased material which, upon application of a biasing force, is elongatable to an elongated, biased length which is at least about 125 percent, that is about one and one quarter, of its relaxed, unbiased length, and which, when elongated to 125 percent of its unbiased length, will recover at least about 90 percent of its elongation within one (1) minute of termination of the elongating force.

A hypothetical example which would satisfy this definition of an elastomeric material would be a one (1) inch sample of a material which is elongatable to at least about 1.25 inches and which, upon being elongated to at least about 1.25 inches and released, will recover to a relaxed length of not more than about 1.025 inches within one (1) minute of termination of the elongating force.

Many elastomeric materials may be elongated by much more than about 25 percent of their relaxed, unbiased length and many of these will recover sufficiently for their utilization in the present invention. For example, some elastomeric materials may be elongated to at least about 50 percent of their relaxed, unbiased length and will recover at least about 75 percent of their elongation, for example at least about 90 percent of their elongation, within one (1) minute of termination of the elongating force. More particularly, some elastomeric materials may be elongated to at least about 100 percent of their relaxed, unbiased length and will recover at least about 75 percent of their elongation, for example at least about 90 percent of their elongation, within one (1) minute of termination of the elongating force. Even more particularly, some elastomeric materials may be elongated to at least about 200 percent of their relaxed, unbiased length and will recover at least about 75 percent of their elongation, for example at least about 90 percent of their elongation, within one (1) minute of termination of the elongating force. Yet even more particularly, some elastomeric materials may be elongated to at least about 300 percent of their relaxed, unbiased length and will recover at least about 75 percent of their elongation, for example at least about 90 percent of their elongation, within one (1) minute of termination of the elongating force.

As used herein the term "recover" refers to the contraction of an elongated material upon termination of the elongating force following elongation of the material by application of the elongating force.

For example, if a material having a relaxed, unbiased length of one (1) inch was elongated 25 percent by stretching to a length of one and one quarter (1.25) inches, the material would have a stretched length that is 125 percent of its relaxed length. If this exemplary stretched material contracted, that is recovered to a length of one and twenty-five thousandths (1.025) inches after termination of the elongating force, the material would have recovered 90 percent (0.225 inch) of its elongation (0.25 inches).

Unless specifically set forth and defined or otherwise limited, the term "polymeric" is used herein to generally include, while not being limited to, homopolymers, copolymers, such as, for example, block, graft, random and alternating copolymers, terpolymers, etc. and blends and modifications thereof. Furthermore, unless otherwise specifically limited, the terms "polymer" or "polymer resin" shall include all possible geometrical configurations of the material. These configurations include, but are not limited to, isotactic, syndiotactic and random symmetries.

Examples of elastomeric polymeric materials include, without limitation, lightly cross-linked natural rubber, block copolymers such as, for example, A-B-A' block copolymers, polyesters, polyurethanes, elastomeric polyolefins and crosslinked dimethylsiloxane.

As used herein the term "elastomeric sheet material" refers to a generally nonporous item formed from an elastomeric polymeric material that can be arranged in generally planar configuration which, in an unapertured state, prior to being modified in accordance with the present invention, has a hydrostatic pressure (hydrohead) of at least about 100 cm of water when measured in accordance with Federal Test Method NO. 5514, standard no. 191A. This term are also intended to include multilayer materials which include at least one such elastomeric polymeric sheet as a layer thereof.

As used herein the term "thin elastomeric polymeric sheet material" refers to an elastomeric polymeric sheet material having an average thickness generally of less than about ten (10) mils. Average thickness is determined by randomly selecting five (5) locations on a given sheet material, measuring the thickness of the sheet material at each location to the nearest 0.1 mil, and averaging the five values (sum of the five values divided by five).

As used herein the term "mesh count" refers to the number which is the product of the number of wires in a wire mesh screen in both the machine (MD) and cross-machine (CD) directions in a given unit area. For example, a wire mesh screen having 100 wires per inch in the machine direction and 100 wires per inch in the cross machine direction would have a mesh count of 10,000 per square inch. As a result of the interweaving of these wires, raised areas are present on both sides of the mesh screen. The number of raised areas on one side of such a wire mesh screen is generally one-half of the mesh count.

As used herein the term "aperture" refers to a generally linear hole or passageway. Aperture is to be distinguished from and does not include holes or passageways having the greatly tortuous path or passageways found in membranes.

As used herein the term "microaperture" refers to an aperture which has an area of less than about 100,000 square micrometers. The area of the microaperture is to be measured at the narrowest point in the linear passageway or hole.

Pressure sensitive valves are valves which are substantially impervious to the passage of water vapor when closed and which open in response to an application of pressure. The pressure sensitive valves of the present invention generally have a maximum functional aperture of less than about 300,000 square micrometers. For example, the pressure sensitive valves may have a maximum functional aperture of less than about 200,000 square micrometers.

Pressure sensitive microvalves are valves which are substantially impervious to the passage of water vapor when closed and which, when opened in response to an application of pressure, have a maximum functional aperture on the order of about 100,000 square micrometers or less.

As used herein the term "substantially impervious to the passage of water vapor" means a valve which will substantially not pass water vapor at one (1) atmosphere.

As used herein the term "maximum functional aperture" refers to the maximum aperture to which a pressure sensitive valve may open while still being able, upon removal of the pressure, to return to a closed configuration which is substantially impervious to the passage of water vapor. That is to say, the maximum aperture of the valve which does not destroy the valve's ability to function as such.

As used herein the term "ultrasonic vibrations" refers to vibrations having a frequency of at least about 20,000 cycles per second. The frequency of the ultrasonic vibrations may range from about 20,000 to about 400,000 cycles per second.

As used herein the term "fluid permeable" refers to a material which has the ability to pass both liquids and gases.

As used herein the term "gas or gaseous permeable" refers to a material which only passes gaseous substances to any significant extent.

As used herein the term "hydrosonics" refers to the application of ultrasonic vibrations to a material where the area of such application is has had a liquid applied thereto to the extent that the liquid is present in sufficient quantity to generally fill the gap between the tip of the ultrasonic horn and the surface of the material.

As used herein the "Sheffield" porosity test refers to TAPPI test number T547, revised May 8, 1989.

### OBJECTS OF THE INVENTION

Accordingly, it is a general object of the present invention to provide a pressure sensitive two-way valve formed from a thin elastomeric polymeric sheet.

It is another general object of the present invention is to provide a pressure sensitive, multi-layer, one-way valve system which includes a thin elastomeric sheet having pressure sensitive two-way valves formed therein.

Still further objects and the broad scope of applicability of the present invention will become apparent to those of skill in the art from the details given hereinafter. However, it should be understood that the detailed description of the presently preferred embodiments of the present invention is given only by way of illustration because various changes and modifications well within the spirit and scope of the invention will become apparent to those of skill in the art in view of this detailed description.

### SUMMARY OF THE INVENTION

In response to the forgoing problems and difficulties encountered by those in the art, we have developed a thin elastomeric polymeric sheet material having at least about 100 pressure sensitive two-way valves per square inch of the sheet material. For example, the thin elastomeric polymeric sheet material may have at least about 1,000 valves per square inch of the sheet material. More particularly, the thin elastomeric polymeric sheet material may have at least about 5,000 valves per square inch of the sheet material. Even more particularly, the thin elastomeric polymeric sheet material may have at least about 20,000 valves per square inch of the sheet material. Yet even more particularly, the thin elastomeric polymeric sheet material may have at least about 90,000 valves per square inch of the sheet material. For example, the thin elastomeric polymeric sheet material may have at least about 160,000 valves per square inch of the sheet material. Of course, as the number of valves per square inch increases, the maximum possible size generally decreases.

The thin elastomeric polymeric sheet material may be formed from any material which meets the definition, contained herein, of an elastomeric material. For example, the thin elastomeric polymeric sheet material may be formed from one or more materials selected from the group including lightly cross-linked natural rubber, block copolymers such as, for example, A-B-A' block copolymers, polyesters, polyurethanes, elastomeric polyolefins and cross-linked dimethylsiloxanes

Because difficulties have been encountered where the elastomeric polymeric sheet is less than about 1 mil thick, it is desirable for the average thickness of the thin elastomeric polymeric sheet material to be at least about 1 mil. For example, the average thickness of the thin elastomeric polymeric sheet material may range from at least about 3 mils to about 8 mils. More particularly, the average thickness of the thin elastomeric polymeric sheet material may range from at least about 3 mil to about 6 mils. Even more particularly, the average thickness of the thin elastomeric polymeric sheet material may range from at least about 4 mil to about 5 mil.

In some embodiments the valves may be microvalves. For example, the maximum functional microaperture of each of the microvalves may generally range from at least about 10 square micrometers to about 100,000 square micrometers. That is, the maximum functional microaperture of each of the microvalves may generally range from at least about 10 square micrometers to about 10,000 square micrometers. More particularly, the maximum functional microaperture of each of the microvalves may generally range from at least about 10 square micrometers to about 5,000 square micrometers. Even more particularly, the maximum functional microaperture of each of the microvalves may generally range from at least about 10 square micrometers to about 1,000 square micrometers.

In some embodiments the valves may be confined to a predesignated area or areas of the thin elastomeric polymeric sheet material.

The valve laden thin elastomeric polymeric sheet material described above may be used as the valve-containing layer in a multilayer one-way valve system. The system, as a whole, includes (1) a valve layer having an outer surface and an inner surface and which is formed from a thin elastomeric polymeric sheet material having at least about 100 pressure sensitive two-way valves per square inch of the elastomeric polymeric sheet material; and (2) a fluid-permeable or gaseous-permeable support layer having an outer surface and an inner surface. The inner surface of the valve layer is joined, in juxtaposed fashion, to the inner surface of the support layer in a manner such that (a) when pressurized fluid exits the inner surface of the support layer, the valves in the valve layer open and allow passage of the fluid through the valve layer; and (b) when pressurized fluid is applied to the outer surface of the valve layer, the support layer prohibits opening of the valves in the valve layer.

The valve layer of the one-way valve system may be formed from any material which meets the definition of elastomeric contained herein. For example, the valve layer may be formed from one or more materials selected from the group including lightly cross-linked natural rubber, block copolymers such as, for example, A-B-A' block copolymers, polyesters, polyurethanes, elastomeric polyolefins and cross-linked dimethylsiloxane.

The support layer of the one-way valve system may be formed from any appropriately fluid permeable material which supplies sufficient resistance to the opening of the valves when pressurized fluid is applied to the outer surface of the valve layer. For example, the support layer may be formed from a material selected from the group including of meltblown web, spunbonded web, a woven fabric, a bonded carded web or an apertured film.

In some embodiments, the multilayer valve system may include additional layers. Of course, it is important that the additional layers be arranged in such a manner as not to interfere with the opening and closing of the valves. Because of this it is usually desirable for the outer surface of the valve layer to be the outermost surface of the system. This configuration allows the valves to freely open away from the applied fluid.

In some embodiments the valves of the one-way valve system may be microvalves. The microvalves may have a maximum functional aperture within any of the ranges, detailed above, for the microvalves of the two-way valve elastomeric sheet.

In some embodiments, the multilayer valve system may include only the support layer and the valve layer.

In some embodiments, the fluid permeable support layer is only permeable to a gaseous substance such as air. In other embodiments, the fluid permeable support layer is permeable to both gaseous and liquid substances such as water and urine.

### THE FIGURES

Figure I is a schematic representation of apparatus which utilizes ultrasonic vibrations to form valves in thin elastomeric polymeric sheet materials.

Figure II is a cross sectional view of the transport mechanism for transporting the thin elastomeric polymeric sheet material to the area where it is subjected to ultrasonic vibrations.

Figure III is a detailed view of the area where the thin elastomeric polymeric sheet material is subjected to ultrasonic vibrations. The area is designated by the dotted circle in Figure I.

Figure IV is a schematic cross-sectional view of a two-way valve formed by the process of the present invention.

Figure V is a schematic cross-sectional view of a one-way valve system which utilizes the two-way valve of Figure IV as a layer thereof.

Figure VI is a photomicrograph of a 4.0 mil thick thin elastomeric polymer sheet of natural rubber which had apertures formed in it while stretched at about 100 percent elongation and which has been returned to its unstretched condition. For comparison purposes, the photomicrograph is accompanied by a photomicrograph, same scale, of the natural rubber prior to processing.

Figure VII is a photomicrograph of the sheet of natural rubber depicted in Figure IV with the sheet stretched to 100 percent elongation to illustrate the presence of the valves in the open configuration. The photomicrograph is accompanied by a scale where each unit represents ten (10) microns (micrometers).

### DETAILED DESCRIPTION OF THE INVENTION

Turning now to the Figures where like reference numerals represent like structure and, in particular to Figure I which is a schematic representation of an apparatus which can carry out the method of the present invention, it can be seen that the apparatus is generally represented by the reference numeral 10. In operation, a supply roll 12 of a thin elastomeric polymeric sheet material 14 which is to be provided with valves is provided. As has been previously stated, the term thin elastomeric polymeric sheet material refers to sheet materials which have an average thickness of about ten (10) mils or less. Additionally, generally speaking the average thickness of the thin elastomeric polymeric sheet material 14 will be at least about 1 mil. For example, the average thickness of the thin elastomeric polymeric sheet 14 material may range from about 3 mils to about 8 mils. More particularly, the average thickness of the thin elastomeric polymeric sheet material 14 may range from about 3 mils to about 6 mils. Even more specifically, the average thickness of the thin elastomeric polymeric sheet material 14 may range from about 4 mil to about 5 mils.

The thin elastomeric polymeric sheet material 14 may be formed from any suitable material meeting the definition of elastomeric contained herein. For example, the thin elastomeric polymeric sheet material may be formed from one or more materials selected from the group including lightly cross-linked natural rubber, block copolymers such as, for example, A-B-A' block copolymers, polyesters, polyurethanes, elastomeric polyolefins and cross-linked dimethylsiloxane.

The thin elastomeric polymeric sheet material 14 is transported to a first nip 16 formed by a first transport roll 18 and a first nip roller 20 by the action of an endless transport mechanism 22 which moves in the direction indicated by the arrow 24. In some embodiments it is necessary for the supply roll 12 to unwind at a slower rate than the first nip roller 20 revolves. This action elongates the material 14 between the supply roll 12 and the nip roller 20. Elongation of the material 14 is necessary with some materials for the process of the present invention to be effective. While no hard and fast rule is known to be applicable, generally speaking, as either or both of the thickness and the elastomeric nature of the material 14 increases, the necessity to elongate increases. The transport mechanism 22 is driven by the rotation of the first transport roller 18 in conjunction with a second transport roller 26 which, in turn, are driven by a conventional power source, not shown.

Figure II is a cross sectional view of the transport mechanism 22 taken along lines A-A in Figure I. Figure II discloses that the transport mechanism 22 includes a heavy duty transport wire mesh screen 28 usually having a mesh count of less than about 400 (i.e. less than a 20 wires per inch MD by 20 wires per inch CD mesh screen if machine direction (MD) and cross machine direction (CD) wire count is the same). Heavy duty mesh wire screens of this type may be made from a variety of materials such as, for example, plastics, nylons or polyesters, and are readily available to those in the art. Located above and attached to the transport screen 28 is an endless flat shim plate 30. The shim plate 30 desirably is formed from stainless steel. However, those of skill in the art will readily recognize that other materials may be utilized. Located above and attached to the shim plate 30 is a fine mesh pattern screen 32 usually having a mesh count of at least about 100 (i.e. at least about 10 wires per inch MD by 10 wires per inch CD mesh screen if MD and CD wire count is the same). Fine mesh wire screens of this type are readily available to those in the art. The fine mesh wire screen 32 has raised areas or knuckles 34 which perform the function of a pattern anvil as will be discussed later.

From the first nip 16 the thin elastomeric polymeric sheet material 14 is transported by the transport mechanism 22 over a tension roll 36 to an area 38 (defined in Figure I by the dotted lined circle) where the thin elastomeric polymeric sheet material 14 is subjected to ultrasonic vibrations.

The assembly for subjecting the thin elastomeric polymeric sheet material 14 to the ultrasonic vibrations is conventional and is generally designated at 40. The assembly 40 includes a power supply 42 which, through a power control 44, supplies power to a piezoelectric transducer 46. As is well known in the art, the piezoelectric transducer 46 transforms electrical energy into mechanical movement as a result of the transducer's vibrating in response to an input of electrical energy. The vibrations created by the piezoelectric transducer 46 are transferred, in conventional manner, to a mechanical. movement booster or amplifier 48. As is well known in the art, the mechanical movement booster 48 may be designed to increase the amplitude of the vibrations (mechanical movement) by a known factor depending upon the configuration of the booster 48. In further conventional manner, the mechanical movement (vibrational energy) is transferred from the mechanical movement booster 48 to a conventional knife edge ultrasonic horn 50. It should be realized that other types of ultrasonic horns 50 could be utilized. For example, a rotary type ultrasonic horn could be used. The ultrasonic horn 50 may be designed to effect yet another boost or increase in the amplitude of the mechanical movement (vibrations) which is to be applied to the thin elastomeric polymeric sheet material 14. Lastly, the assembly includes an actuator 52 which includes a pneumatic cylinder, not shown. The actuator 52 provides a mechanism for raising and lowering the assembly 40 so that the tip 54 of the ultrasonic horn 50 can apply tension to the transport mechanism 22 upon the assembly 40 being lowered. It has been found that it is necessary to have some degree of tension applied to the transport mechanism 22 upon the lowering of the assembly for proper application of vibrational energy to the thin sheet material 14 to form apertures in the thin elastomeric polymeric sheet material 14. One desirable aspect of this tensioned arrangement is that the need to design a finely toleranced gap between the tip 54 of the horn 50 and the raised areas or knuckles 34 of the fine mesh wire screen 32 is not necessary.

Figure III is a schematic representation of the area 38 where the ultrasonic vibrations are applied to the thin elastomeric polymeric sheet material 14. As can be seen in Figure III, the transport mechanism 22 forms an angle 56 with the tip 54 of the ultrasonic horn 50. While some aperturing will occur if the angle 56 is as great as 45 degrees, it has been found that it is desirable for the angle 56 to range from about 5 degrees to about 15 degrees. For example, the angle 56 may range from about 7 to about 13 degrees. More particularly, the angle 56 may range from about 9 to about 11 degrees.

Figure III also illustrates that the transport mechanism 22 is supported from below by the first tension roll 36 and a second tension roll 58. Positioned somewhat prior to the tip 54 of the ultrasonic horn 50 is a spray nozzle 60 which is configured to apply a fluid 62 to the surface of the thin elastomeric polymeric sheet material 14 just prior to the sheet material 14 being subjected to ultrasonic vibrations by the tip 54 of the ultrasonic horn 50. The fluid 62 desirably may be selected from the group including one or more of water, mineral oil, a chlorinated hydrocarbon, ethylene glycol or a solution of 50 volume percent water and 50 volume percent 2 propanol. For example, in some embodiments the chlorinated hydrocarbon may be selected from the group including 1,1,1 trichloroethane or carbon tetrachloride. It should be noted that the wedge-shaped area 64 formed by the tip 54 of the ultrasonic horn 50 and the transport mechanism 22 should be subjected to a sufficient amount of the fluid 62 for the fluid 62 to act as both a heat sink and a coupling agent for the most desirable results. Positioned below the transport mechanism 22 in the area where the tip 54 of the ultrasonic horn 50 is located is a fluid collection tank 66. (See figure I.) The fluid collection tank 66 serves to collect fluid 62 which has been applied to the surface of the thin elastomeric polymeric sheet material 14 and which has either been driven through the sheet material 14 and/or the transport mechanism 22 or over the edges of the transport mechanism 22 by the action of the vibrations of the tip 54 of the ultrasonic horn 50. Fluid 62 which is collected in the collection tank 66 is transported by tubing 68 to a fluid holding tank 70.

Figure I illustrates that the fluid holding tank 70 contains a pump 72 which, by way of additional tubing 74, supplies the fluid 62 to the fluid spray nozzle 60. Accordingly, the fluid 62 may be re-cycled for a considerable period of time.

While the mechanism of action may not be fully understood and the present application should not be bound to any particular theory or mechanism of action, it is believed that the presence of the fluid 62 in the wedge-shaped area 64 during operation of the ultrasonic horn 50 accomplishes two separate and distinct functions. First, the presence of the fluid 62 allows the fluid 62 to act as a heat sink which allows the ultrasonic vibrations to be applied to the thin elastomeric polymeric sheet material 14 without the thin elastomeric polymeric sheet material 14 being altered or destroyed as by melting. Secondly, the presence of the fluid 62 in the wedge-shaped area 64 allows the fluid 62 to act as a coupling agent in the application of the vibrations from the ultrasonic horn 50 to the thin elastomeric polymeric sheet material 14.

It has been discovered that the action of the ultrasonic horn 50 on the thin elastomeric polymeric sheet material 14 apertures the thin elastomeric polymeric sheet material 14 in spite of the fact that there are no fibers to re-arrange to form apertures as was the case in Mitchell et al.. The apertures are punched through the thin elastomeric polymeric sheet material 14 in the pattern of the raised areas or knuckles 34 of the fine mesh wire pattern screen 32. Generally, the number of apertures produced will be equal to the number of raised areas or knuckles 34 on the upper surface of the fine mesh wire screen 32. That is, the number of apertures will generally be one-half the mesh count of a given area of pattern screen 32. For example, if the pattern screen 32 is 100 wires per inch MD by 100 wires per inch CD, the total number of knuckles or raised areas 34 on one side of the pattern wire, per square inch, 32 will be 100 times 100 divided by 2. This equals 5,000 apertures per square inch. For a 200 wires per inch by 200 wires per inch mesh pattern screen 32 the calculation yields 20,000 apertures per square inch. Depending somewhat on the thickness of the thin elastomeric polymeric sheet material 14, at a mesh count of about 90,000 (300 wires per inch MD by 300 wires per inch CD) the wires are so thin as to allow the knuckles 34 on both sides to aperture the thin elastomeric polymeric sheet material 14 if sufficient force is applied. Thus, a 300 wires per inch MD by 300 wires per inch CD mesh screen yields 90,000 apertures per square inch; for a 400 wires per inch MD by 400 wires per inch CD mesh--160,000 apertures per square inch. Of course the MD and CD wire count of the wire mesh screen does not have to be the same.

Because the aperturing process is used to form apertures in a material which is elastomeric, the apertures close back up as a result of the recovery of the thin elastomeric polymeric sheet material 14 after removal of the wire knuckle from the aperture. Where the sheet material 14 is in the stretched configuration during processing, removal of the stretching force also aides in the closing up of the apertures. Accordingly, Figure IV illustrates that a two-way pressure sensitive valve 74 is formed by each aperture because the apertures will open back up in response to an application of a pressurized fluid 76 to either side of the thin elastomeric sheet material 14. The valves 74 open up in response to the pressurized fluid 76 by being stretched or elongated out of the plane of the thin elastomeric sheet material 14 to form a generally conical valve having a truncated apex 78 and a larger base 80.

In some embodiments of the present invention it may be desirable to provide a one-way valve system. Figure V illustrates that such a system may be formed by joining a fluid permeable support sheet material 82, in overlapping juxtaposed configuration, to a thin elastomeric polymeric material 14 which has been provided with valves 74. The fluid permeable support sheet 82 may be formed from any appropriately fluid-permeable sheet material which supplies the support necessary, discussed below, for this embodiment. For example, the fluid permeable support sheet may be formed from a material selected from the group including of meltblown web, spunbonded web, a woven fabric, a bonded carded web or an apertured film. In some embodiments the fluid permeable support sheet 82 may be only permeable to gaseous substances while in other embodiments the fluid permeable support sheet 82 may be permeable to both liquid substances and gaseous substances. The fluid permeable support sheet material 82 must have sufficient strength so that, when its inner surface 84 is joined to the inner surface 86 of the thin elastomeric sheet material 14, the support sheet 82 provides sufficient support to the inner surface 84 of the thin elastomeric sheet material 14 so that when pressurized fluid 76 is applied to the outer surface 88 of the thin elastomeric polymeric sheet material 14, the valves 74 will no be allowed to move out of the plane of the thin elastomeric polymeric sheet material 14 and will remain closed. Conversely, when pressurized fluid 76 is applied to the outer surface of the fluid permeable support sheet 82, the fluid will pass through the support sheet 82 and will apply pressure to the inner surface of the thin elastomeric polymeric sheet material 14. Because there is no support sheet joined to the outer surface 88 of the thin elastomeric polymeric sheet material 14, the valves 74 will open outwardly, away from the plane of the elastomeric sheet material 14. Thus, the system provides a one-way valve arrangement.

It should also be noted that the number of valves formed may also vary with the number of ultrasonic vibrations to which the thin elastomeric polymeric sheet material 14 is subjected per unit area for a given period of time. This factor may be varied in a number of ways. For example, the number and size of the valves will vary somewhat with the line speed of the thin elastomeric polymeric sheet material 14 as it passes underneath the tip 54 of the ultrasonic horn 50. Generally speaking, as line speed increases, first the size of the valves decreases and then the number of valves decreases. As the number of valves decreases the less the pattern of apertures/valves resembles the pattern of raised areas 34 on the pattern screen 32. The range of line speeds that usually yields valves varies with the elastomeric polymeric material utilized to form the thin elastomeric polymeric sheet material 14 and the material used as the fluid 62. For lightly cross-linked natural rubber having a thickness of about 4.0 mils, typical line speeds which usually yield valves for a wide variety of fluids range from about 7 to about 10 feet per minute. For example, if water is used as the fluid with lightly cross-linked natural rubber typical line speeds which usually yield valves range from about 7 to about 10 feet per minute. It is believed that, to some extent, the variations in the number of valves formed and the size of the valves occurs due to the minute variations in the height of the raised areas or knuckles 34 of the fine mesh pattern screen 32. It should be noted that the fine mesh pattern screens used to date have been obtained from conventional everyday sources such as a hardware store. It is also believed that if a pattern screen 32 could be created where all of the raised areas 34 of the screen 32 were of exactly the same height these variations would only occur in uniform fashion with variations of line speed.

As was stated above, the area or size of each of the valves formed will also vary with the parameters discussed above. The area of the valves will also vary with the area of the raised areas of the pattern anvil such as the knuckles 34 on the fine mesh wire screen 32. It is believed that the type of material used in forming the thin elastomeric polymeric sheet material 14 will also vary the area of the valves formed if all other parameters are maintained the same. For example, the softer the thin elastomeric polymeric sheet material 14, the easier it is to push the thin elastomeric polymeric sheet material 14 through the raised areas of the fine mesh pattern screen 32. Because the raised areas (knuckles) on the fine mesh screen are generally pyramidal in shape, the deeper the raised area penetrates the thin elastomeric polymeric sheet material 14, the larger the aperture. Of course, the height of the raised areas must be greater than the thickness of the thin sheet material 14 for apertures to be formed and the degree of excess necessary may vary with the type of elastomeric polymeric sheet material 14 to be valved. Generally speaking, the more stretchable the material, the greater the height of the raised areas 34 must exceed the thickness of the elastomeric sheet material 14.

In some embodiments it may be necessary to subject the thin elastomeric polymeric sheet material 14 to multiple passes through the apparatus 10 in order to aperture/valve the thin sheet material 14. However, two or more passes through the apparatus 10, with the thin elastomeric polymeric sheet material 14 being aligned in the same configuration with respect to the pattern anvil, yields valves. Alternatively, the fine mesh wire diameter size may be increased with the consequent decrease in mesh count. Increasing the wire diameter size of the fine mesh screen 32 increases the likelihood that valves will be formed.

Another feature of the present invention is the fact that the valves can be formed in a predesignated area or areas of the thin elastomeric polymeric sheet material 14. This can be accomplished in a number of ways. For example, the thin elastomeric polymeric sheet material 14 may be subjected to ultrasonic vibrations only at certain areas of the sheet material 14, thus, microvalving would occur only in those areas. Alternatively, the entire thin elastomeric polymeric sheet material 14 could be subjected to ultrasonic vibrations with the pattern anvil having raised areas 34 only at certain locations and otherwise being flat. Accordingly, the thin elastomeric polymeric sheet material would be valved only in those areas which corresponded to areas on the pattern anvil having raised areas.

It should also be noted that some limitation exists in the number of valves which can be formed in a given thin elastomeric polymeric sheet material 14 on a single application of vibrational energy, i.e. a single pass through the apparatus 10 if a wire mesh screen is used as the pattern anvil. This follows from the fact that, as was stated above, the height of the raised areas 34 must exceed the thickness of the thin elastomeric polymeric sheet material 14 in conjunction with the fact that, generally as the mesh count increases the height of the raised areas or knuckles decreases. In such situations, if the number of valves desired per unit area is greater than the number which can be formed in one pass through the apparatus, multiple passes are necessary with the alignment of the thin elastomeric polymeric sheet material 14 with respect to the raised ares being altered or shifted slightly on each pass.

In some embodiments the valves may be microvalves. For example, the maximum functional microaperture of each of the microvalves may range from at least about 10 square micrometers to about 100,000 square micrometers. For example, the maximum functional microaperture of each of the microvalves may generally range from at least about 10 square micrometers to about 10,000 square micrometers. More particularly, the maximum functional microaperture of each of the microvalves may generally range from at least about 10 square micrometers to about 1,000 square micrometers. Even more particularly, the maximum functional microaperture of each of the microvalves may generally range from at least about 10 square micrometers to about 100 square micrometers.

A number of important observations about the process may now be made. For example, it should be understood that the presence of the fluid 62 is highly important to the present inventive process which uses the fluid as a coupling agent. Because a coupling agent is present, the apertures are punched in the thin sheet material 14 as opposed to being formed by melting. Additionally, the presence of the shim plate 30 or its equivalent is necessary in order to provide an anvil mechanism against which the thin elastomeric polymeric sheet material 14 may be worked, that is apertured, by the action of the tip 54 of the ultrasonic horn 50. Because the vibrating tip 54 of the ultrasonic horn 50 is acting in a hammer and anvil manner when operated in conjunction with the heavy duty wire mesh screen 28/shim plate 30/fine wire mesh 32 combination, it should be readily recognized that a certain degree of tension must be placed upon the transport mechanism 22 by the downward displacement of the ultrasonic horn 50. If there is little or no tension placed upon the transport mechanism 22, the shim plate 30 cannot preform its function as an anvil and microvalving generally does not occur. Because both the shim plate 30 and the fine mesh pattern wire 32 form the resistance that the ultrasonic horn 50 works against, they are collectively referred herein as a pattern anvil combination. It should be easily recognized by those in the art that the function of the pattern anvil can be accomplished by other arrangements than the heavy duty wire mesh screen 28/shim plate 30/fine mesh screen 32 combination. For example, the pattern anvil could be a flat plate with raised portions acting to direct the aperturing force of the ultrasonic horn 50. Alternatively, the pattern anvil could be a cylindrical roller having raised areas. If the pattern anvil is a cylindrical roller with raised areas, it is desirable for the pattern anvil to be wrapped or coated with or made from a resilient material. Where the pattern anvil is a mesh screen the resiliency is provided by the fact that the screen is unsupported directly below the point of application of ultrasonic vibrations to the mesh screen.

The invention will now be discussed with regard to specific examples which will aid those of skill in the art in a full and complete understanding thereof.

### EXAMPLE I

A sheet of 4.0 mil thick lightly cross-linked natural rubber having the trade designation Softlastic Rubber TS 3000 was obtained from the J.P. Stevens Comapny of Northampton, MA. and was cut into a length of about 18 inches and a width of about 10 inches. Because part of the rubber was used to secure it to the jig in the stretching processes, discussed below, only about 15 inches of length of the natural rubber was apertured. The hydrohead of such natural rubber prior to hydrosonic treatment has been measured as being at least 137 inches of water. (This is the highest value obtainable with the equipment used.) The sample was subjected to hydrosonic treatment in accordance with the present invention.

A model 1120 power supply obtained from the Branson Company of Danbury, Connecticut, was utilized. This power supply, which has the capacity to deliver 1,300 watts of electrical energy, was used to convert 115 volt, 60 cycle electrical energy to 20 kilohertz alternating current. A Branson type J4 power level control, which has the ability to regulate the ultimate output of the model 1120 power supply from 0 to 100%, was connected to the model 1120 power supply. In this example, the power level control was set at 100%. The actual amount of power consumed was indicated by a Branson model A410A wattmeter. This amount was about 900 watts.

The output of the power supply was fed to a model 402 piezoelectric ultrasonic transducer obtained from the Branson Company. The transducer converts the electrical energy to mechanical movement. At 100% power the amount of mechanical movement of the transducer is about 0.8 micrometers.

The piezoelectric transducer was connected to a mechanical movement booster section obtained from the Branson Company. The booster is a solid titanium metal shaft with a length equal to one-half wave length of the 20 kilohertz resonant frequency. Boosters can be machined so that the amount of mechanical movement at their output end is increased or decreased as compared to the amount of movement of the transducer. In this example the booster increased the amount of movement and has a gain ratio of about 1:2.5. That is, the amount of mechanical movement at the output end of the booster is about 2.5 times the amount of movement of the transducer.

The output end of the booster was connected to an ultrasonic horn obtained from the Branson Company. The horn in this example is made of titanium with a working face of about 9 inches by about 1/2 inch. The leading and trailing edges of the working face of the horn are each curved on a radius of about 1/8 inch. The horn step area is exponential in shape and yields about a two-fold increase in the mechanical movement of the booster. That is, the horn step area has about a 1:2 gain ratio. The combined increase, by the booster and the horn step area, in the original mechanical movement created by the transducer yields a mechanical movement of about 4.0 micrometers.

The forming table arrangement included a small forming table which was utilized to transport and support the sheet of natural rubber to be apertured. The forming table included two 2-inch diameter idler rollers which were spaced about 12 inches apart on the surface of the forming table. A transport mesh belt encircles the two idler rollers so that a continuous conveying or transport surface is created. The transport mesh belt is a square weave 20 x 20 mesh web of 0.020 inch diameter plastic filaments. The belt is about 10 inches wide and is raised above the surface of the forming table.

The transducer/booster/horn assembly, hereinafter the assembly, is secured in a Branson series 400 actuator. When power is switched on to the transducer, the actuator, by means of a pneumatic cylinder with a piston area of about 4.4 square inches, lowers the assembly so that the output end of the horn contacts the sheet of natural rubber which is to be apertured. The actuator also raises the assembly so that the output end of the horn is removed from contact with the sheet of natural rubber when power is switched off.

The assembly is positioned so that the output end of the horn is adapted so that it may be lowered to contact the transport mesh belt between the two idler rollers. An 8-inch wide 0.005-inch thick stainless steel shim stock having a length of about 60 inches was placed on the plastic mesh transport belt to provide a firm support for a pattern screen which is placed on top of the stainless steel shim. In this example the pattern screen is a 120 by 120 wires per inch MD and CD wire size weave stainless steel screen. The sheet of natural rubber which was to be apertured was then stretched 100 percent and held in the stretched position by a jig laid on the wire with the jug being fastened to the wire with tape.

The forming table arrangement also included a fluid circulating system. The circulating system includes a fluid reservoir tank, a fluid circulating pump which may conveniently be located within the tank, associated tubing for transporting the fluid from the tank to a slotted boom which is designed to direct a curtain of fluid into the juncture of the output end of the horn and sheet of natural rubber which is to be valved.

In operation, the assembly was positioned so that the output end of the horn was at an angle of from about 10 to 15 degrees to the sheet of natural rubber to be valved. Accordingly, a wedge shaped chamber was formed between the output end of the horn and the sheet of natural rubber to be valved. It is into this wedge shaped chamber that the fluid, in this example water at room temperature, is directed by the slotted boom.

It should be noted that the actuator was positioned at a height to insure that, then the assembly is lowered, the downward movement of the output end of the horn is stopped by the tension of the transport mesh before the actuator reaches the limit of its stroke. In this example, actuating pressure was adjusted to 7 pounds per square inch as read on a pressure gauge which is attached to the pneumatic cylinder of the actuator. This adjustment results in a total downward force of 30.8 pounds. (7 psi times 4.4 square inches of piston area equals 30.8 pounds of force.)

The sequence of operation was (1) the fluid pump was switched on and the area where the output end of the horn was to contact the stretched sheet of natural rubber was flooded with water; (2) the transport mesh conveyor system was switched on and the stretched natural rubber started moving at 4 feet per minute; and (3) power to the assembly was supplied and the assembly was lowered so that the output end of the horn contacted the sheet of stretched natural rubber while the sheet continued to pass under the output end of the horn until the end of the sample was reached. The reading on the A410A wattmeter during the process is an indication of the energy required to maintain maximum mechanical movement at the output end of the horn while working against the combined mass of the fluid, the sheet of natural rubber, the pattern wire, the shim stock, and the transport wire.

This example yielded an elastomeric sheet material having a maximum aperture density of about 7,200 apertures per square inch when in the 100 percent stretched configuration. Accordingly, the maximum valve density of was about 14,400 valves per square inch upon removal of the elongating force. The valves had an a maximum functional aperture area in excess of 100,000 square micrometers.

Using the Sheffield Porosity Test, the opening pressure for the valves was at a water height of about 4.2 centimeters on the gauge indicating an opening pressure of about 0.004 atmosphere. This value is an average of two measurements.

The water vapor transmission rate of the valved sheet was about 562 grams of water per square meter per day. The wvtr of the elastomeric sheet prior to being valved was measured as about 40 grams of water per square meter per day. This indicates that the valves open and close as water vapor pressure builds up. This value is an average of two measurements.

The hydrohead of the valved sheet was measured as being about 98 centimeters of water. (one measurement) Thus, the valved material maintained a significant portion of its strength.

Figure VI is two photomicrographs of the natural rubber sheet used in Example I. The top photomicrograph is of the sheet prior to processing. The bottom photomicrograph is of the sheet after processing and with the 100 percent stretching force having been removed. No evidence of the presence of the valves is present in the processed photomicrograph.

Figure VII is a photomicrograph of the natural rubber sheet valved in accordance with Example I and stretched to 100 percent to illustrate the presence of the valves. The photomicrograph is accompanied by a scale where each unit represents ten (10) microns (micrometers).

### EXAMPLE II

The process of example I was repeated with the exception that the natural rubber was processed six (6) times with the pattern screen being an 18 by 23 wire having a braided cross-hatch.

This example yielded an elastomeric sheet material having a maximum aperture density of about 1,800 apertures per square inch when in the 100 percent stretched configuration. Because of the braiding the number of apertures exceeds one-half the mesh count. Accordingly, the maximum valve density of was about 3,600 valves per square inch upon removal of the elongating force. The valves had an a maximum functional aperture area in excess of 300,000 square micrometers.

Using the Sheffield Porosity Test, the opening pressure for the valves was at a water height of about 4.2 centimeters on the gauge indicating an opening pressure of about 0.004 atmosphere. This value is an average of two measurements.

The water vapor transmission rate of the valved sheet was about 343 grams of water per square meter per day. The wvtr of the elastomeric sheet prior to being valved was measured as about 40 grams of water per square meter per day. This indicates that the valves open and close as water vapor pressure builds up. This value is an average of two measurements.

The hydrohead of the valved sheet was measured as being about 24 centimeters of water. (one measurement) Thus, the valved material maintained a good portion of its strength.

### EXAMPLE III

The process of example I was repeated with the exception that the test material was a 5 mil thick sheet of dimethylsiloxane obtained under the trade designation Silastic which was not stretched during processing and which was processed eight (8) times. The pattern screen being an 18 by 23 wire having a braided cross-hatch. Additionally, the line speed was 4 feet per minute and the watts consumed were about 800-1,000.

This example yielded an elastomeric sheet material having a maximum aperture density of about 1,700 valves per square inch. Because of the braiding the number of apertures exceeds one-half the mesh count. The valves had an a maximum functional aperture area in excess of about 100,000 square micrometers.

Using the Sheffield Porosity Test, the opening pressure for the valves was at a water height of about 13 centimeters on the gauge indicating an opening pressure of about 0.0124 atmosphere.

The water vapor transmission rate of the valved sheet was about 396 grams of water per square meter per day. The wvtr of the material in an unprocessed state was 218 grams per square meter per day. This indicates that the valves open and close as water vapor pressure builds up. This value is an average of two measurements.

The hydrohead of the valved sheet was measured as being about 20 centimeters of water. (one measurement) The hydrohead of this material prior to treatment was measured as being in excess of 137 centimeters of water. Thus, the valved material maintained a good portion of its strength.

### EXAMPLE IV

The process of example I was repeated with the exception that the sheet was a 1.0 mil thick sheet of a polyether polyurethane obtained from the J.P Stevens Co. under the trade designation XPR-824 which was not stretched during processing. Additionally, the line speed was about 3.8 feet per minute, the watts consumed were about 800 and the actuating pressure was about 10 pounds per square inch.

This example yielded an elastomeric sheet material having a maximum valve density of about 7,200 valves per square inch. The valves had an a maximum functional aperture area in excess of about 100,000 square micrometers.

Using the Sheffield Porosity Test, the opening pressure for the valves was at a water height of about 19 centimeters on the gauge indicating an opening pressure of about 0.0181 atmosphere.

The water vapor transmission rate of the valved sheet was about 566 grams of water per square meter per day. The wvtr of the elastomeric sheet prior to being valved was measured as about 447 grams of water per square meter per day. This indicates that the valves open and close as water vapor pressure builds up. This value is an average of two measurements.

The hydrohead of the valved sheet was measured as being about 37 centimeters of water. (one measurement) The hydrohead of this material prior to processing was measured as being in excess of 137 centimeters of water. Thus, the valved material maintained a good portion of its strength.

The uses to which the valved or microvalved elastomeric polymeric sheet material of the present invention may be put are numerous. For example, the microvalved thin elastomeric polymeric material may be utilized as an outer cover for a disposable diaper, a feminine care pad product cover, a food wrap, a pressure release system where ever it is desirable to maintain one atmosphere of pressure in a narrow range.

It is to be understood that variations and modifications of the present invention may be made without departing from the scope of the invention. For example, in some embodiments the use of multiple ultrasonic horns aligned abreast or sequentially may be desirable. It is also to be understood that the scope of the present invention is not to be interpreted as limited to the specific embodiments disclosed herein, but only in accordance with the appended claims when read in light of the foregoing disclosure.

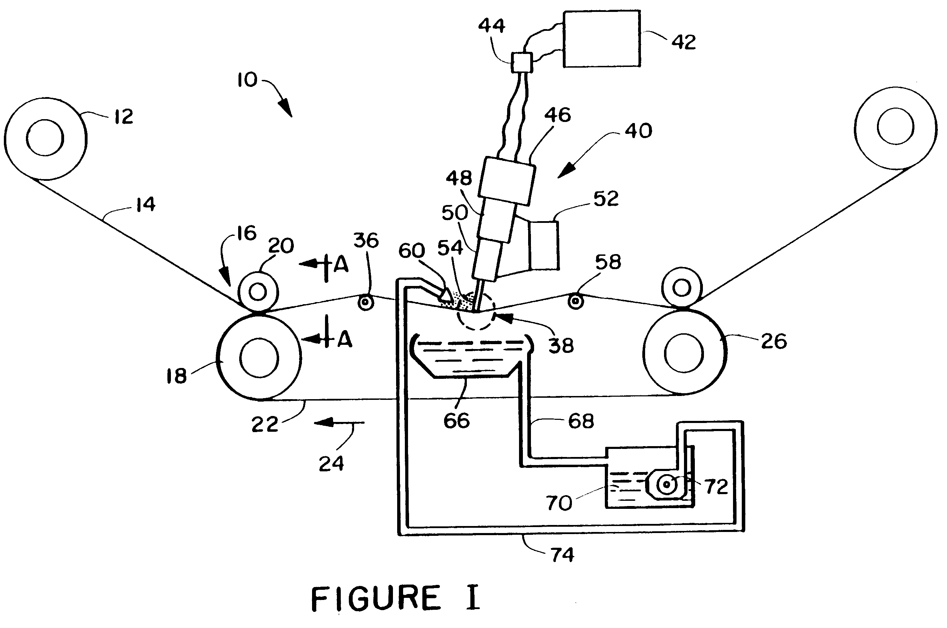

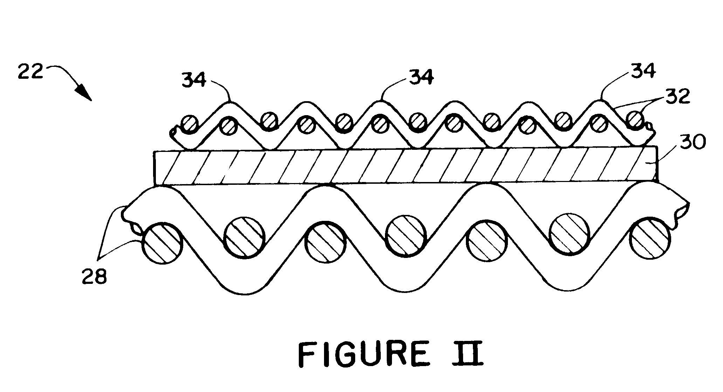

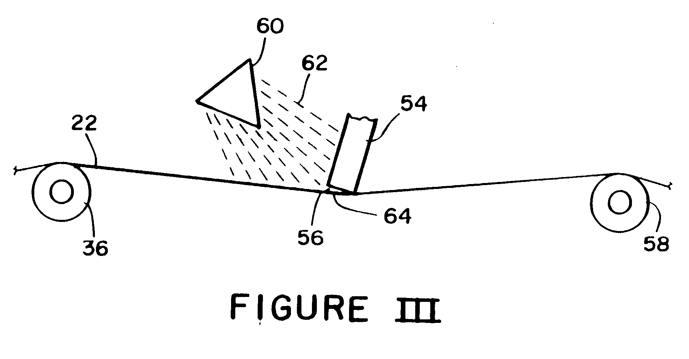

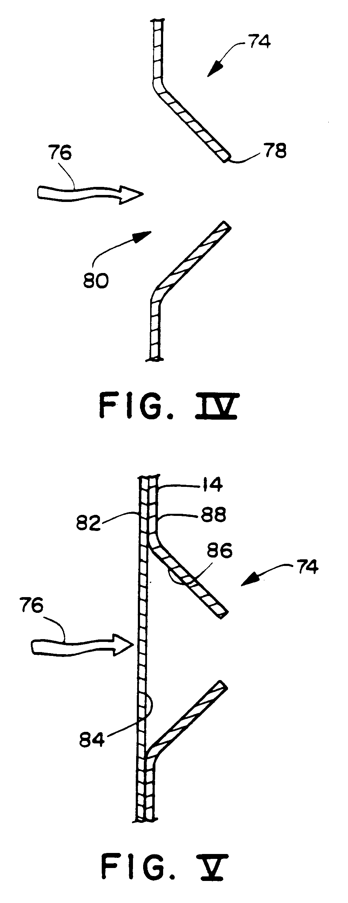

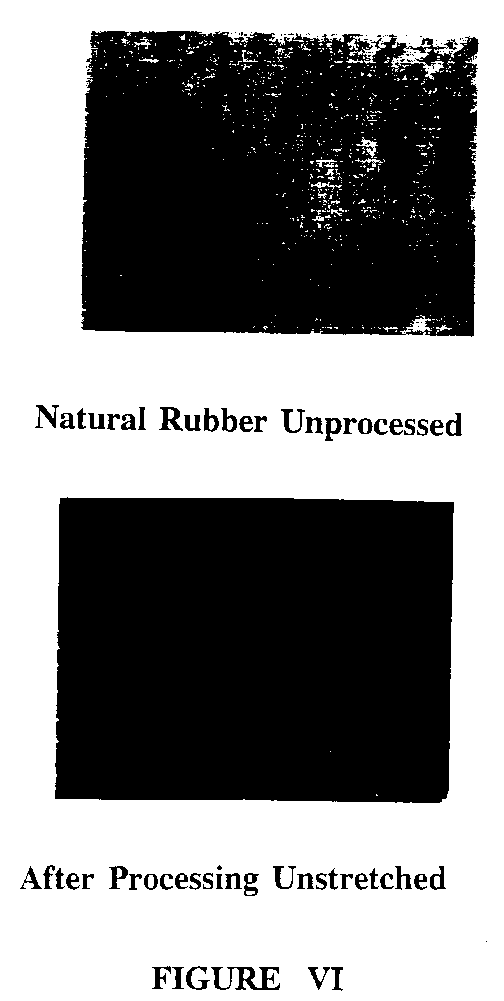

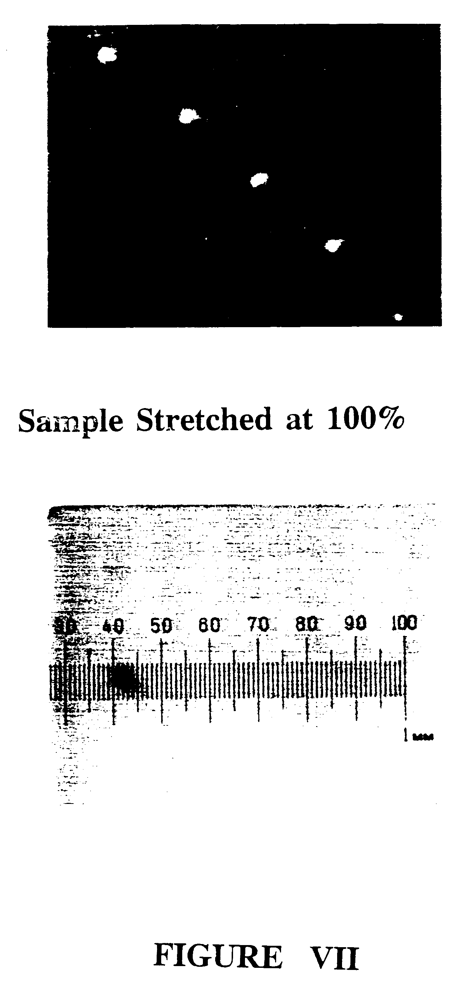

## Claims

1. A thin elastomeric polymeric sheet material having an average thickness of at least about 1 mil and at least about 100 pressure sensitive two-way valves per square inch of the sheet material.

2. The thin elastomeric polymeric sheet material of claim 1, wherein the sheet material has at least about 1,000 valves per square inch of the sheet material.

3. The thin elastomeric polymeric sheet material of claim 1, wherein the sheet material has at least about 5,000 valves per square inch of the sheet material.

4. The thin elastomeric polymeric sheet material of claim 1, wherein the sheet material has at least about 20,000 valves per square inch of the sheet material.

5. The thin elastomeric polymeric sheet material of claim 1, wherein the sheet material has at least about 90,000 valves per square inch of the sheet material.

6. The thin elastomeric polymeric sheet material of claim 1, wherein the sheet material has at least about 160,000 valves per square inch of the sheet material.

7. The thin elastomeric polymeric sheet material of claim 1, wherein the thin elastomeric polymeric sheet material is be formed from one or more materials selected from the group including lightly cross-linked natural rubber, block copolymers such as, for example, A-B-A' block copolymers, polyesters, polyurethanes, elastomeric polyolefins and cross-linked dimethylsiloxanes.

8. The thin elastomeric polymeric sheet material of claim 1, wherein the average thickness of the sheet material is from at least about 3 mils to at least about 8 mils.

9. The thin elastomeric polymeric sheet material of claim 1, wherein the average thickness of the sheet material is from at least about 3 mil to about 6 mils.

10. The thin elastomeric polymeric sheet material of claim 1, wherein the average thickness of the sheet material is from at least about 4 mils to about 5 mils.

11. The thin elastomeric polymeric sheet material of claim 1, wherein the valves are microvalves.

12. The thin elastomeric polymeric sheet material of claim 11, wherein the maximum functional aperture area of each of the microvalves generally ranges from at least about 10 square micrometers to about 100,000 square micrometers.

13. The thin elastomeric polymeric sheet material of claim 11, wherein the maximum functional aperture area of each of the microvalves generally ranges from at least about 10 square micrometers to about 10,000 square micrometers,

14. The thin elastomeric polymeric sheet material of claim 11, wherein the maximum functional aperture area of each of the microvalves generally ranges from at least about 10 square micrometers to about 5,000 square micrometers.

15. The thin elastomeric polymeric sheet material of claim 11, wherein the maximum functional aperture area of each of the microvalves generally ranges from at least about 10 square micrometers to about 1,000 square micrometers.

16. The thin elastomeric polymeric sheet material of claim 1, wherein the valves are confined to a predesignated area or areas of the thin elastomeric polymeric sheet material.

17. A thin elastomeric sheet material having a thickness of about 4 to about 5 mils, said sheet material having:
a microvalve density of at least about 100,000 microvalves per square inch; and
wherein the maximum functional aperture area of each of the microvalves ranges generally from greater than about 10 square micrometers to less than about 1,000 square micrometers.

18. A multilayer one-way valve system comprising:
a valve layer having an outer surface and an inner surface, the valve layer comprising:
a thin elastomeric polymeric sheet material having a thickness of at least about 1 mil and at least about 100 pressure sensitive two-way valves per square inch of the elastomeric polymeric sheet material; and
a support layer having an outer surface and an inner surface, the support layer comprising:
a fluid permeable sheet material; and
wherein the inner surface of the valve layer is joined, in juxtaposed fashion, to the inner surface of the support layer in a manner where:
when pressurized fluid exits the inner surface of the support layer, the valves in the valve layer open and allow passage of the fluid through the valve layer; and
when pressurized fluid is applied to the outer surface of the valve layer, the support layer prohibits opening of the valves in the valve layer.

19. The one-way valve system of claim 18, wherein the valve layer is formed from one or more materials selected from the group consisting of lightly cross-linked natural rubber, block copolymers such as, for example, A-B-A' block copolymers, polyesters, polyurethanes, elastomeric polyolefins and cross-linked dimethylsiloxanes.

20. The one-way valve system of claim 18, wherein the support layer is formed from a material selected from the group consisting of meltblown web, spunbonded web, a woven fabric, a bonded carded web or an apertured film.

21. The one-way valve system of claim 18, wherein the multilayer valve system where the outer surface of the valve layer is an outermost surface of the system.

22. The one-way valve system of claim 21, wherein the multilayer valve system consists of two layers.

23. The one-way valve system of claim 18, wherein the valves are microvalves.

24. The one-way valve system of claim 23, wherein the thin elastomeric polymeric sheet material has at least about 1,000 microvalves per square inch of the sheet material.

25. The one-way valve system of claim 23, wherein the thin elastomeric polymeric sheet material has at least about 5,000 microvalves per square inch of the sheet material.

26. The one-way valve system of claim 23, wherein the thin elastomeric polymeric sheet material has at least about 20,000 microvalves per square inch of the sheet material.

27. The one-way valve system of claim 23, wherein the thin elastomeric polymeric sheet material has at least about 90,000 microvalves per square inch of the sheet material.

28. The one-way valve system of claim 23, wherein the thin elastomeric polymeric sheet material has at least about 160,000 microvalves per square inch of the sheet material.

29. The one-way valve system of claim 18, wherein the fluid permeable support layer is permeable to gaseous substances but not to liquid substances.

30. The one-way valve system of claim 18, wherein the fluid support layer is permeable to gaseous substances and liquid substances.
